# EUROPEAN PATENT APPLICATION

(11) **EP 3 594 957 A1**
(43) Date of publication of application: **15.01.2020**
(21) Application number: 18182627.2
(22) Date of filing: 10.07.2018
(51) Int. Cl.: G16H 10/65

(54) **PATIENT LABELING DEVICE**

(71) Applicant: Dalin Tzu Chi Hospital, Buddhist Tzu Chi Medical Foundation, 62247 Chiayi County (TW)
(72) Inventor: CHENG, Po-Liang, 62247 Chiayi County (TW)
(74) Representative: Viering, Jentschura & Partner mbB Patent- und Rechtsanwälte

(57) **Abstract**

A patient labeling device is worn on the patient (1), such that when the patient labeling device is sensed to be connected with the terminal device (2), the terminal device (2) is allowed to read or edit the patient information (4) stored in the cloud database (3). The patent labeling device includes a main body (10) having a sense label (14). The sense label (14) is sensed to be connected with the terminal device (2) and provides an information transaction function between the sense label (14) and the terminal device (2). Therefore, the identity of the patient (1) is efficiently identified.

## Description

### BACKGROUND OF THE INVENTION

### 1. Field of the Invention:

The present invention relates to identity identification fields, and more particularly, to a patient labeling device.

### 2. Description of the Related Art:

In a large scale accident, medical staff from various hospitals arrive the scene of the accident for rescuing a large amount of patients. A medical personnel has to efficiently identify the identity of each patient, so as to provide different treatment procedures according to the different injury levels of the patients.

Regarding a conventional procedure for handling the large amount of patients, the medical staff usually identify the patient identities and carry out the medical tag registration manually, and provide treatments according to different medical examination levels. However, manually identifying the patient identities not only prolongs the rescue duration, but also easily cause mistaken identification and registrations. As a result, medical malpractice might occur due to the misidentification of patients.

Also, patient information acquired by manual medical examination and medical tag registration are unable to be shared for all medical staff. Therefore, a single patient might undergo the same identification process by different medical personnel, delaying the rescuing procedure and causing a waste of resource.

Further, the initial identity identification and medical examination are manually registered by use of medical tag. If the information of the medical tag fails to be delivered to the hospital together with the patient, the medical staff in the hospital have to carry out the identity identification and medical examination again. Also, the medical staff are unable to know if the patient has underwent an initial treatment at the scene of the accident, such that the medical staff in the hospital have to carry out a confirmation and treatment again, causing a waste of medical resource.

### SUMMARY OF THE INVENTION

For improving the issues above, a patient labeling device is disclosed. When the terminal device sense to be connected with the sense label on the main body, the terminal device is allowed to read or edit the patient information. Therefore, the identity and the treatment status of the patient is efficiently identified, achieving a highly efficient medical treatment.

A patient labeling device in accordance with an embodiment of the present invention is worn by a patient, such that when the patient labeling device is sensed to be connected with a terminal device, the terminal device is allowed to read or edit the patient information stored in a cloud database. The patient labeling device comprises a main body having a sense label, wherein the sense label is identified by the terminal device and is able to carry out an information transaction function with the terminal device.

With such configuration, when an accident occurs, medical staff are able to wear the main body on the patient. With the sense label disposed on the main body, when the medical staff senses the sense label on the main body through the terminal device, the medical staff are able to identify the patient identity and confirm the injury status of the patient, so as to establish a patient information in the cloud database. If the patient has received an initial treatment, such treatment is also taken into record in the patient information. Therefore, the medical staff in the subsequent stage are able to efficiently identify the patient identity and confirm the injury and treatment status of the patient, achieving a highly efficient treatment process.

In an embodiment of the present invention, the surface of the main body is covered with a color layer. Therefore, the medical staff are able to apply different color layers upon the main body according to different injury levels of the patient, thus informing medical staff in the subsequent stage about the priority of treatment needed by different patients.

### BRIEF DESCRIPTION OF THE DRAWINGS

**Fig. 1** is a block diagram illustrating the connection of the patient labeling device in accordance with an embodiment of the present invention.
**Fig. 2** is a perspective view of the main body of the patient labeling device in accordance with an embodiment of the present invention.
**Fig. 3** is an enlarged sectional view of **Fig. 2** **of** the patient labeling device in accordance with an embodiment of the present invention.
**Fig. 4** is a schematic view illustrating the operation status of patient labeling device in accordance with an embodiment of the present invention.

### DETAILED DESCRIPTION OF THE INVENTION

The aforementioned and further advantages and features of the present invention will be understood by reference to the description of the preferred embodiment in conjunction with the accompanying drawings where the components are illustrated based on a proportion for explanation but not subject to the actual component proportion.

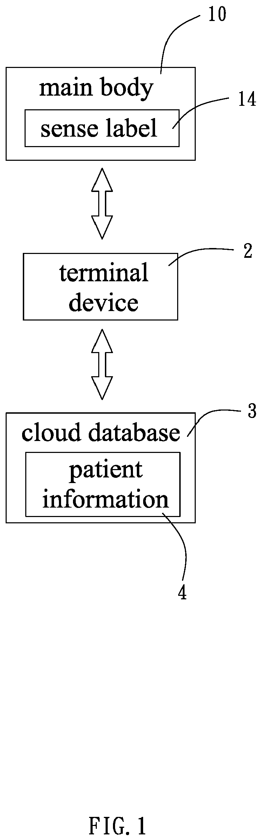

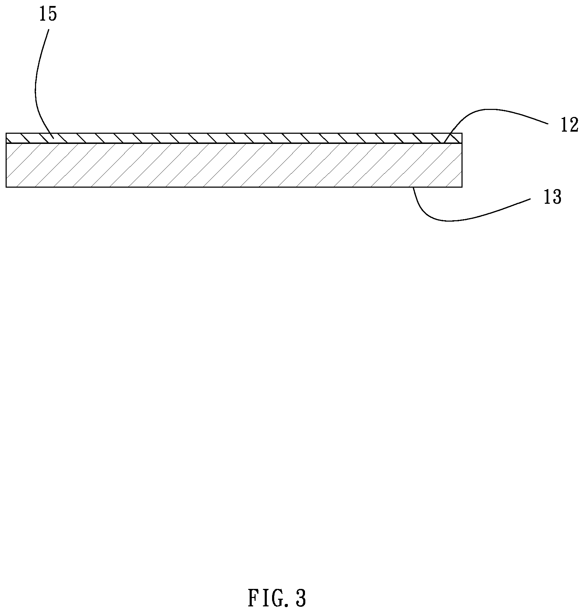

Referring to **Fig. 1** to **Fig. 4****,** a patient labeling device in accordance with an embodiment of the present invention is worn on the patient **1.** When the patient labeling device is sensed to be connected with the terminal device **2,** the terminal device **2** is allowed to read or edit the patient information **4** store in the cloud database **3.** In an embodiment of the present invention, the cloud database **3** is established on a cloud server. At least one medical personnel **5** is allowed to access the cloud database **3** by used of the terminal device **2** through the internet. The medical personnel **5** is able to edit the patient information **4** in the cloud database **3** by the terminal device **2.** In an embodiment of the present invention, the terminal device **2** is a portable device, such as a smart phone or a tablet computer. The patient information **4** is chosen from the group consisting of text, image, video, and a combination thereof.

The patient labeling device in accordance with an embodiment of the present invention comprises a main body **10.**

The main body **10** is formed in an elongate rectangular strip shape, with an end side **11** disposed on two ends of the main body **10.** In an embodiment of the present invention, when the two end sides **11** of the main body **10** are connected with each other, the main body **10** is able to be circled into a wristband. The main body **10** has a front face **12** and an opposite rear face **13,** wherein the front face **12** faces outward, and the rear face **13** faces the skin of the patient **1** and disposed in adjacent to the hand portion **6** of the patient **1.**

The main body **10** has a sense label **14** disposed on the front face **12** of the main body **10** between the two side ends **11** of the main body **10.** The sense label **14** is allowed to be sensed by the terminal device **2** and provides an information transaction function between the sense label **14** and the terminal device **2.** The sense label **14** is an NFC (near field communication) label.

Also, the front face **12** of the main body **10** is covered with a color layer **15,** wherein the color layer **15** is allowed to be covering or not covering the sense label **14.** In an embodiment of the present invention, the color layer **15** does not cover the sense label **14.**

The color layer **15** is able to be present in different colors, such as red, yellow, green, black, or other colors, such that different colors of the color layer **15** illustrates the different injury levels of the patient **1.** For example, the most seriously injured patients **1** to the most minor patients **1** are presented orderly from red, yellow, green, and black color, respectively. Therefore, the medical personnel **5** is aware of the treatment priority of each patient **1.**

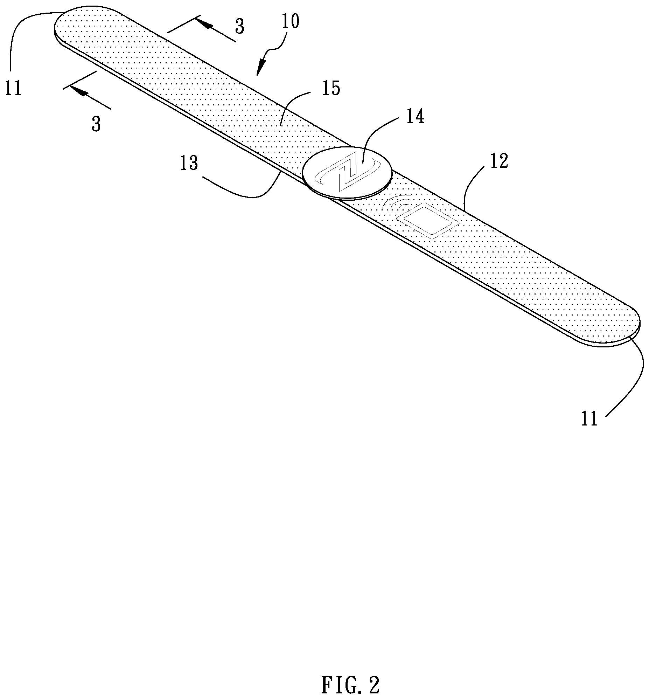

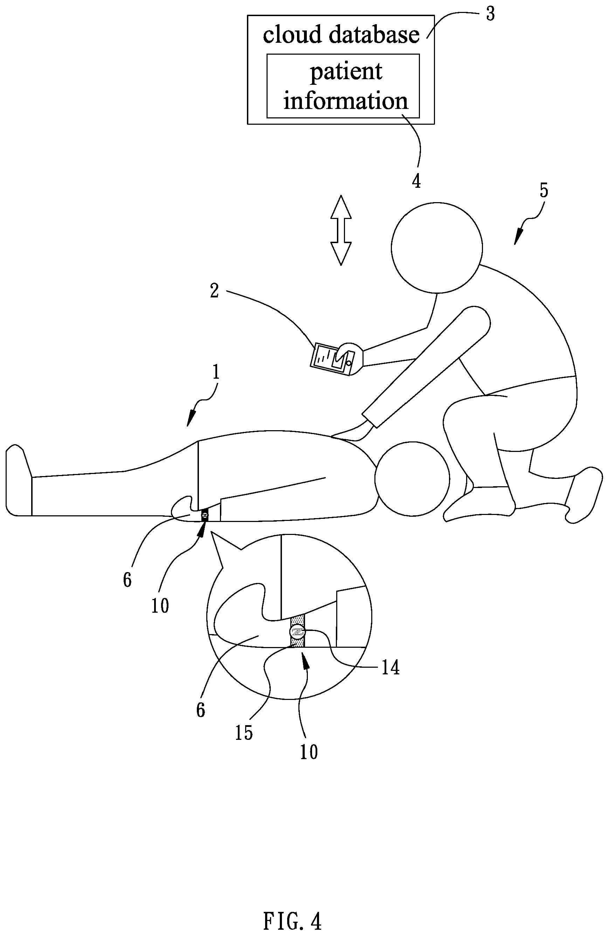

Referring to **Fig. 2** and **Fig. 4****,** when a large scale accident occurs with a plurality of patients **1** existing in the scene of the accident, medical personnel **5** wears the main body **10** on each patient **1** at the scene, respectively. When the identity information of the patient **1** is being confirmed, the terminal device **2** is connected with the sense label **14** through the near field communication, and a patient information **4** is established in the cloud database **3.** Also, during the medical examination, the injury level and the initial treatment status of the patient **1** at the scene are established in the patient information **4.**

Next, when each patient **1** is delivered to the hospital for further treatment, medical personnel **5** carries out the near field communication between the terminal device **2** and the sense label **14** of the main body **10** for reading the patient information **4** of the cloud database **3.** Therefore, the identity and initial treatment status of the patient **1** are effectively and timely acquired, facilitating the further treatment process of the patient **1.**

Also, the medical personnel **5** at the scene of the accident is allowed to provide the main body **10** with different colored color layers **15,** such that the medical personnel **5** in the subsequent treatment stage is allowed to identify the injury level of different patients **1,** so as to provide a proper treatment priority.

Although particular embodiments of the invention have been described in detail for purposes of illustration, various modifications and enhancements may be made without departing from the spirit and scope of the invention. Accordingly, the invention is not to be limited except as by the appended claims.

## Claims

1. A patient labeling device which is worn on a patient (1), such that when the patient labeling device is sensed to be connected with a terminal device (2), the terminal device (2) is allowed to read or edit a patient information (4) stored in a cloud database (3), the patent labeling device comprising:
a main body (10) having a sense label 14, the sense label (14) sensed by the terminal device (2) to be connected with the terminal device (2) and provided an information transaction between the sense label (14) and the terminal device (2).

2. The patient labeling device of claim 1, wherein a surface of the main body (10) is covered by a color layer (15).

3. The patient labeling device of claim 2, wherein the main body (10) comprises a front face (12), and the color layer (15) covers the front face (12).

4. The patient labeling device of claim 3, wherein the sense label (14) is disposed on the front face (12).

5. The patient labeling device of claim 4, wherein the sense label (14) is not covered by the color layer (15).

6. The patient labeling device of claim 1, wherein the main body (10) is formed in an elongate rectangular strip shape.

7. The patient labeling device of claim 6, wherein the main body (10) has two side ends (11) disposed at two ends of the main body (10), such that when the two side ends (11) are connected with each other, the main body (10) is circled into a wristband.

8. The patient labeling device of claim 7, wherein the sense label (14) is disposed between the two side ends (11) of the main body (10).

9. The patient labeling device of claim 1, wherein the sense label (14) is an NFC (near field communication) label.
